# EUROPÄISCHE PATENTSCHRIFT

(11) **EP 2 170 438 B1**
(45) Veröffentlichungstag und Bekanntmachung des Hinweises auf die Patenterteilung: **09.08.2017**
(21) Anmeldenummer: 08759290.3
(22) Anmeldetag: 19.06.2008
(51) Int. Cl.: A61M 5/30, A61M 5/178, A61M 5/20, A61M 5/31, A61M 5/315

(54) **EINWEGINJEKTOR MIT HANDBETÄTIGBAREM KOLBEN**
DISPOSABLE INJECTOR WITH MANUALLY ACTUATED PISTON
INJECTEUR UNE VOIE À PISTON ACTIONNÉ MANUELLEMENT

(30) Priorität: 24.07.2007 DE 102007034871
(43) Veröffentlichungstag der Anmeldung: 07.04.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Siemund, Volker
(86) Internationale Anmeldenummer: PCT/EP2008/004951
(87) Internationale Veröffentlichungsnummer: WO 2009/012855

(56) Entgegenhaltungen:
- EP-A- 1 336 419
- US-A- 3 557 784
- US-A- 4 185 628
- US-A1- 2001 051 789
- US-A1- 2003 105 430
- US-A1- 2003 139 706
- US-A1- 2005 273 048

## Beschreibung

Die Erfindung betrifft einen nadelfreien Einweginjektor mit einem Gehäuse, in dem oder an dem-jeweils zumindest bereichsweise - mindestens ein mechanischer Federenergiespeicher, mindestens eine - zumindest zeitweise wirkstoffbefüllbare - Zylinder-Kolben-Einheit, mindestens ein Kolbenbetätigungsstempel und mindestens eine Auslöseeinheit angeordnet ist, wobei der Federenergiespeicher mindestens ein vorgespanntes Federelement umfasst, wobei zumindest ein Teil des Kolbenbetätigungsstempels zwischen dem Federenergiespeicher und dem Kolben der Zylinder-Kolben-Einheit positioniert ist, wobei der Kolben der Zylinder-Kolben-Einheit - bei vorgespanntem Federelemente - über eine Pumpstange separat bewegbar ist und wobei der Kolbenbetätigungsstempel eine zentrale Bohrung hat, die von der Pumpstange durchquert wird.

Aus der US 2001/0051789 A1 ist u.a. ein derartiger Injektor bekannt. Er hat einen federvorgespannten Kolbenbetätigungsstempel, der beim Betätigen des Injektors auf eine starre Kombination aus Kolben und Kolbenstange des in der Zylinder-Kolben-Einheit geführten Kolbens wirkt. Die einteilige Kolbenstange ist nach hinten so weit verlängert, dass sie über das hintere Gehäuseende des Injektors übersteht. Mittels des hinteren Endes der Kolbenstange kann der Kolben separat von Hand bewegt werden. Der Innenraum des Zylinders der Zylinder-Kolben-Einheit ist hier nicht steril verschließbar, auch wird bei der Injektion die ganze Kolbenstange mitbewegt.

Die US 2003/0105430 A1 beschreibt einen komplizierten, asymmetrisch aufgebauten Einwegnadelinjektor mit eingebautem Nadelschutz und einer fest integrierten Rückholstange. Mit der Rückholstange kann der Kolben der Medikamentenkammer manuell bewegt werden. Beim Auslösen des Injektionsvorganges wird die Rückholstange durch eine plastische Verformung ausgekuppelt. Auch hier ist die Medikamentenkammer nicht steril verschließbar.

Der vorliegenden Erfindung liegt daher die Problemstellung zugrunde, einen modular aufgebauten Einweginjektor zu entwickeln, dessen Zylinder-Kolben-Einheit im eingebauten Zustand befüllbar ist. Dieser Injektor weist bei geringer Baugröße nur wenige Bauteile auf und gewährleistet bei einfacher Handhabung eine sichere Lagerung und Funktion. Auch im manuell befülltem Injektor ist die Injektionslösung bis zu zwei Jahren steril lagerbar.

Diese Problemstellung wird mit den Merkmalen des Hauptanspruchs gelöst. Dazu ist die Pumpstange am Kolben oder an einer Kolbenstange des Kolbens lösbar ankuppelbar. Der Zylinder der Zylinder-Kolben-Einheit weist ein rückwärtiges Dichtelement auf, das den zwischen dem Kolben und dem Zylinder gelegenen Zylinderinnenraum steril verschließt.

Mit der Erfindung wird hier ein nadelfreier Einmalinjektor vorgestellt, dessen Zylinder-Kolben-Einheit bei gespanntem Federspeicher durch eine manuelle Pumpbewegung befüllt werden kann. U.a. wird dazu von außen her eine Pumpstange in den im Injektor angeordneten Kolben der Zylinder-Kolben-Einheit eingekuppelt, so dass mittels der Pumpstange der Kolben bewegt werden kann. Nach dem ordnungsgemäßen Befüllen kann die Zylinder-Kolben-Einheit wieder mit einem sterilen Stopfen steril verschlossen werden. Ggf. wird die Pumpstange ausgekuppelt und aus dem Gehäuse herausgezogen. Durch eine besondere Anordnung der Dichtelemente in der Zylinder-Kolben-Einheit bleibt die Befüllung steril verschlossen.

Weitere Einzelheiten der Erfindung ergeben sich aus den Unteransprüchen und den nachfolgenden Beschreibungen schematisch dargestellter Ausführungsbeispiele.
- Figur 1:: Einweginjektor mit zwei Druckstäben und pumpfähigem Kolben;
- Figur 2:: wie Figur 1, jedoch mit handbefülltem Zylinder;
- Figur 3:: wie Figur 1, jedoch entsichert, betätigt und ohne Pumpstange (fiktiver Zustand);
- Figur 4:: wie Figur 2, jedoch nach dem Medikamentenausstoß;
- Figur 5:: Einweginjektor mit zwei in Sperrstellung verformten Druckstäben;
- Figur 6:: wie Figur 5, jedoch beladen mittels einer Injektionsnadel;
- Figur 7:: wie Figur 6, jedoch entsichert und betätigt (fiktiver Zustand);
- Figur 8:: wie Figur 7, jedoch mit entleertem Zylinder;
- Figur 9:: wie Figur 5, jedoch um 90° um die Injektormittellinie geschwenkt;
- Figur 10:: Dimetrische Ansicht zu Figur 9, jedoch ohne Etikett;
- Figur 11:: Einweginjektor in Sperrstellung mit zwei Zughaken und pumpfähigem Kolben;
- Figur 12:: Ausschnittvergrößerung zu Figur 5;
- Figur 13:: alternative Ausschnittvergrößerung zu Figur 5;
- Figur 14:: Ausschnittvergrößerung zu Figur 10.

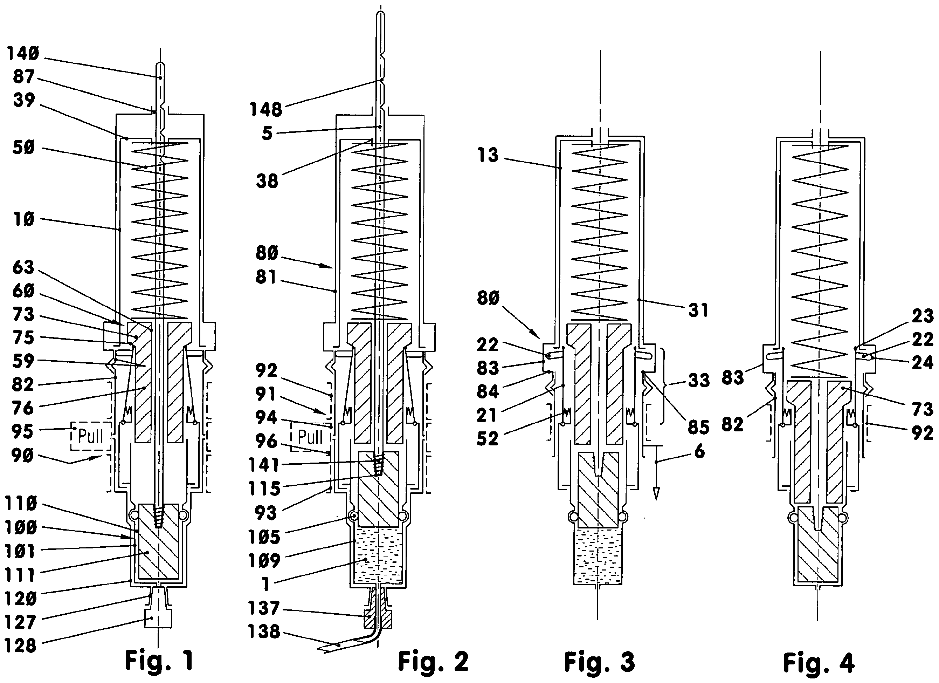

Die Figuren 1 bis 4 zeigen eine vereinfachte Prinzipskizze eines Einweginjektor-Typs mit einem dauergeladenen Federenergiespeicher in drei verschiedenen Auslösezuständen. Der gezeigte Einweginjektor besteht aus einem Gehäuse (10), einer z.B. mit einer Injektionslösung vorbefüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (90) angeordnet. Die Zylinder-Kolben-Einheit (100) ist vorn über eine Verschlusskappe (120) in Kombination mit einem Stopfen (128) verschlossen. Die Zylinder-Kolben-Einheit (100) hat einen Kolben (111), der mittels einer separaten Pumpstange (140) im Zylinder (101) bewegt werden kann.

Das Gehäuse (10) ist ein topfförmiger, unten offener Hohlkörper mit obenliegendem Boden (39). Im mittleren Bereich, dem Mantelbereich (31), hat das Gehäuse (10) z.B. zwei einander gegenüberliegende fensterartige Durchbrüche (33), vgl. Figur 3. Am unteren Rand des einzelnen Durchbruchs (33) ist jeweils ein Druckstab (21) gelenkig gelagert. Der Boden (39) hat eine zentrale Bohrung (38).

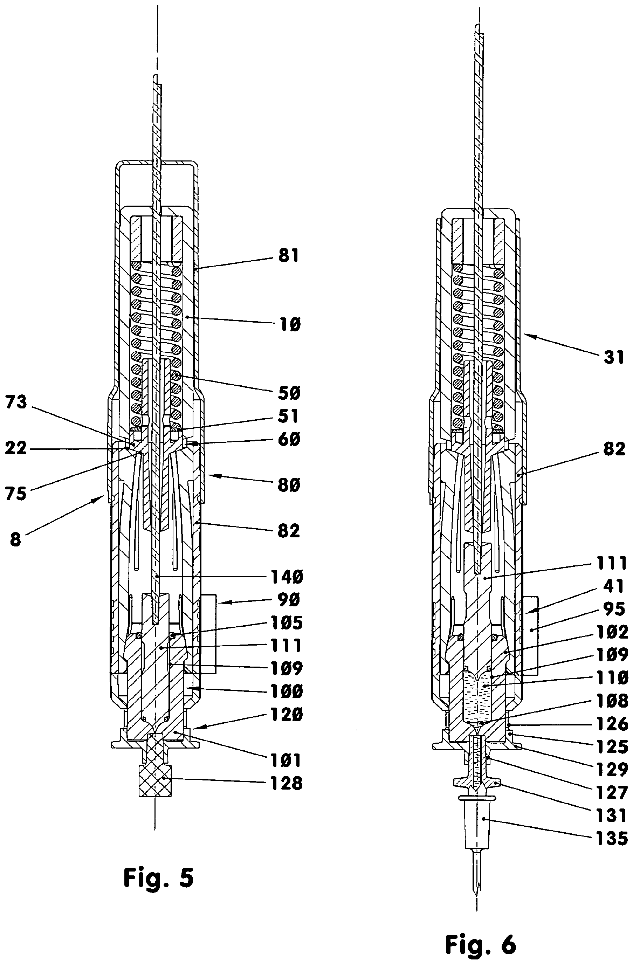

Die Druckstäbe (21) sind hier nur beispielhaft in Schwenkgelenken angeordnet und über Federelemente (52) am Gehäuse (10) abgestützt. Die Federelemente (52) drücken die Stützstäbe (21) zumindest annähernd radial nach außen gegen das Auslöseelement (82), vgl. Figur 1 bis 4. Dort liegen sie über Nocken (22) am Auslöseelement (82) an. Die Nocken (22) können dabei z.B. auch 5 bis 20 Millimeter unterhalb des jeweiligen freien oberen Endes der Druckstäbe (21) liegen. Sind die Druckstäbe (21) am Gehäuse (10) angeformt, vgl. Figuren 5 bis 8, so federn sie als elastische Biegebalken (28) nach außen.

Die beiden auf Druck belasteten Druckstäbe (21) halten den Kolbenbetätigungsstempel (60) an dessen Stempelteller (73) in seiner vorgespannten Lage, vgl. Figur 1. Dazu stützen sich die Druckstäbe (21) mit ihren Abstützflächen (23) am Stempelteller (73) ab. Die Größe der jeweiligen Kontaktfläche zwischen einer Abstützfläche (23) und der entsprechenden Stelle am Stempelteller (73) liegt im Bereich von 2 bis 20 mm².

Auf der der Mittellinie (5) abgewandten Seite weist jeder Druckstab (21) an seinem Nocken (22) eine Anlagefläche (24) auf.

Im unteren Bereich des Gehäuses (10) befinden sich Halteelemente zur Befestigung der Zylinder-Kolben-Einheit (100).

Die Zylinder-Kolben-Einheit (100) besteht im Ausführungsbeispiel aus einem, mit einer Injektionslösung (1) befüllbaren, transparenten Zylinder (101), in dem ein Kolben (111) in der vorderen Position sitzt. Der Zylinder (101) hat eine Zylinderinnenwandung (109), die nach hinten z.B. in einer Ringnut endet. In der Ringnut sitzt ein radial dicht am Kolben (111) anliegendes Dichtelement (105). Das Dichtelement (105) schließt den leeren, nur mit dem Kolben (111) großteils ausgefüllten Zylinderinnenraum (110) steril ab. Hinter der Ringnut weitet sich der Zylinder (101) so weit auf, dass ein rückwärts bewegter Kolben (111) die Wandung des aufgeweiteten Bereichs nicht kontaktieren kann.

Der Kolben (111) hat an seiner Rückseite eine z.B. zentrale, kegelstumpfmantelförmige Ausnehmung (115), in der die Pumpstange (140) mittels eines Kegelgewindes (141) eingeschraubt ist. Die Pumpstange (140) kann mit geringem Kraftaufwand vom Kolben (111) gelöst werden.

Oberhalb des Kolbens (111) ist im Gehäuse (10) der Kolbenbetätigungsstempel (60) z.B. so angeordnet, dass er den Kolben zwar nicht berührt, jedoch mit seinem unteren Ende z.B. im oberen Bereich des Zylinders (101) seitlich geführt wird. Der Kolbenbetätigungsstempel (60) hat eine zentrale Bohrung (63), die von der Pumpstange (140) durchquert wird.

Nach Figur 1 ist die untere Hälfte des Gehäuses (10) von dem hülsenartigen Auslöseelement (82) umgeben. Das Auslöseelement (82) ist auf der radialen Außenfläche (13) des Gehäuses (10) längsverschiebbar gelagert. Es endet rückwärtig mit einer scharfen Kante (85). Unterhalb der Kante (85) berühren nach Figur 1 die Nocken (22) mit ihren außen liegenden Anlageflächen (24) sichernd die Innenwandung (59) des Auslöseelements (82).

Beispielsweise in der Nähe der Kante (85) ist am Auslöseelement (82) eine Auslösekappe (81) befestigt, die das hintere Ende des Gehäuses (10) vollständig umgibt. Dort hat sie eine zentrale Bohrung (87) zum Durchführen der Pumpstange (140). Die Auslösekappe (81) umfasst eine umlaufende Aufweitung (83), in der beim Auslösen des Injektors die Nocken (22) aufgenommen werden. Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Druckstäbe (21) mit ihren Nocken (22) aufnehmen kann. Die Innenkontur der Aufweitung (83) ist z.B. ein Kanal mit einer Rücksprungflanke (84), die hier eine zur Mittellinie (5) des Injektors normale Ebene darstellt.

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101). Die untere Stirnfläche des Kolbenschiebers (76) wirkt direkt auf den Kolben (111).

Der obere Bereich des Kolbenbetätigungsstempels (60), also der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 130, vorzugsweise 120 Winkelgrade beträgt. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

An das vordere Ende des Auslöseelements (82) schließt sich die Verschlusskappe (120) an. Letztere umhüllt den unteren Teil der Zylinder-Kolben-Einheit (100). Die Verschlusskappe (120) weist an der vorderen Stirnseite eine Adapteröffnung (127) mit z.B. einem Luer-Innenkegel auf, die mittels eines außenkegligen Stopfens (128) steril verschlossen ist. Die Verschlusskappe (120) ist hier auf dem unteren Bereich des Gehäuses (10) gelagert. Sie und das hülsenartige Auslöseelement (82) sind zumindest bereichsweise mit einem Klebeetikett (91) ummantelt. Das Klebeetikett (91) besteht aus einem Hauptteil (92), einer Abreißbanderole (94) und einem Kappenteil (93). Die Abreißbanderole (94) ist mit den Etikettteilen (92, 93) durch eine Sollbruchstelle (96), z.B. eine Perforation oder eine durchgehende Materialdünnstelle, verbunden. Die Abreißbanderole (94) ist hierbei über der zwischen dem Auslöseelement (82) und der Verschlusskappe (120) gelegenen Montagefuge angeordnet. Sie endet in einer abstehenden Abreißfahne (95).

Es kann auch ein vollflächiges Klebeetikett verwendet werden, das im Bereich der die Teile (82) und (120) trennenden Montagefuge ein reißfestes Zugmittel enthält. Das Zugmittel, z.B. ein Faden, ein Kunststoffstreifen, ein dünner Draht oder dergleichen steht an einer Seite über das Etikett über. Beim Abziehen des Zugmittels wird das Etikett im Bereich der Montagefuge gezielt aufgetrennt.

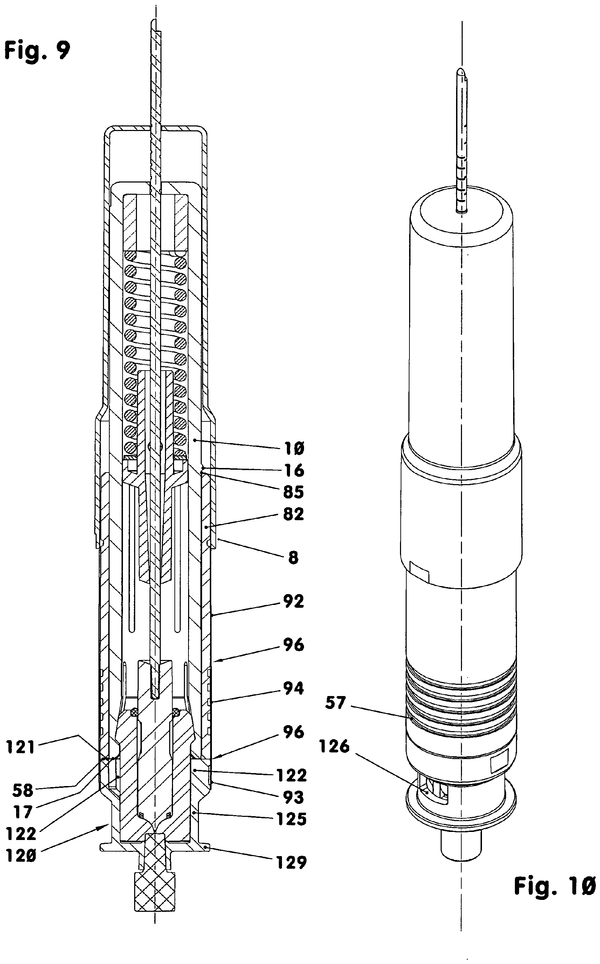

Um den Einweg-Injektor benutzen zu können, muss zunächst die Zylinder-Kolben-Einheit (100) befüllt werden. Dazu wird der Stopfen (128) aus der Adapteröffnung (127) entfernt und durch einen Schlauchadapter (137) ersetzt. Letzterer weist einen Pumpschlauch (138) auf. Mit dem Pumpschlauch (138) wird aus einer geöffneten Medikamentenampulle die Injektionslösung (1) durch das Zurückziehen des Kolbens (111) mittels der Pumpstange (140) in den Zylinder (101) gesaugt. Die Ansaugmenge der Injektionslösung (1) kann über die an der Pumpstange (140) befindende Skala (148) kontrolliert werden. Der Ansaugvorgang wird abgeschlossen, indem die ggf. in den Zylinder (101) angesaugten Gasblasen in bekannter Weise z.B. durch ein geringfügiges Zurückschieben des Kolbens (111) - bei nach oben gehaltener Verschlusskappe (120) - entfernt werden. Zur Kontrolle dienen zwei in der Verschlusskappe (120) angeordnete Fenster (126), vgl. Figur 10. Nun wird beispielsweise die Pumpstange (140) aus der Ausnehmung (115) des Kolbens (111) herausgedreht und aus dem Gehäuse (10) herausgezogen.

Zum Entsichern des Injektors wird die Abreißbanderole (94) abgezogen, so dass die Klebeverbindung zwischen der Verschlusskappe (120) und dem Auslöseelement (82) aufgehoben ist. Die Verschlusskappe (120) und der Schlauchadapter (137) werden abgezogen. Der Einweg-Injektor wird auf der Injektionsstelle positioniert. Nun kann das Auslöseelement (82) in Richtung der Zylinder-Kolben-Einheit (100) verschoben werden, vgl. Figur 3. Bei diesem Vorgang gleitet das Auslöseelement (82) auf der Außenwandung (13) des Gehäuses (10) linear nach unten, also in Richtung der Injektionsstelle. Die Anlageflächen (24) der Druckstäbe (21) rutschen über die Kante (85) und springen unter der Kraft des Federelements (50) entsichernd radial nach außen in die Aufweitung (83). Der Kolbenbetätigungsstempel (60) schnellt ungehindert nach unten, vgl. Figur 4. Der Zylinder (100) wird entleert.

Anstelle einer linearen Gleitbewegung des Auslöseelements (82) auf dem Gehäuse (10) kann auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Die Figuren 5 bis 10 zeigen eine Ausführungsform des in den Figuren 1 bis 4 beschriebenen Prinzips. Hier ist das tragende Bauteil ein einteiliges Gehäuse (10). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

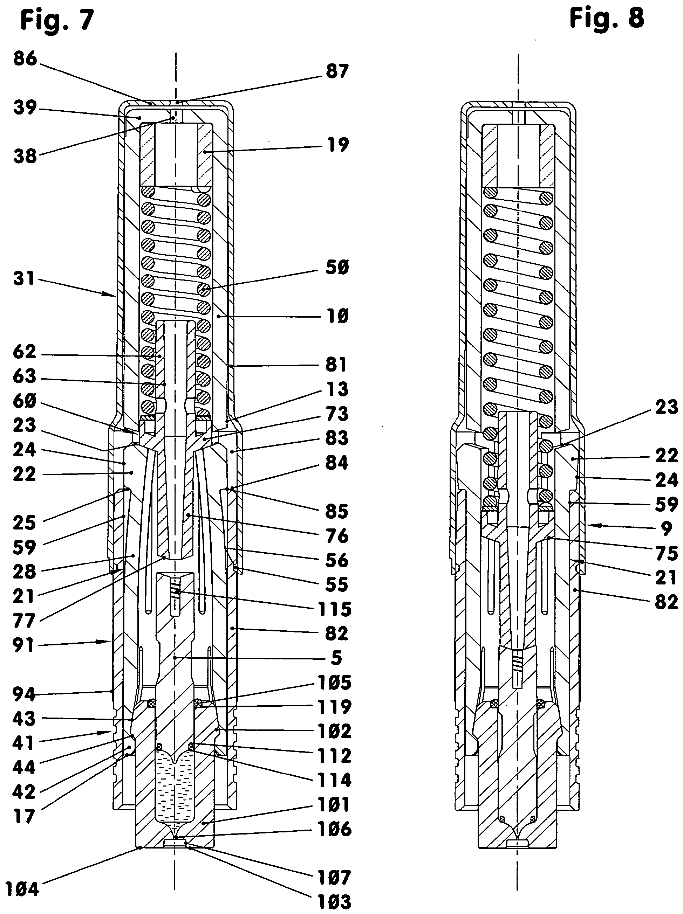

Der im wesentlichen rohrförmige Mantelbereich (31) ist oben durch einen z.B. ebenen Boden (39) mit integrierter Bohrung (38) verschlossen, vgl. Figur 7. In der unteren Hälfte des Mantelbereichs (31) befinden sich zwei einander gegenüberliegende, angeformte Druckstäbe (21). Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab seitlich und oben umgibt. Der Druckstab (21) hat auf ca. 80% seiner Länge die Wandstärke und die Krümmung der Wandung des Gehäuses (10). Dieser Bereich hat u.a. auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren. In der Figur 8 ist der Druckstab (21) im unverformten Zustand dargestellt.

Das hier obere freie Ende des einzelnen Druckstabs (21) wird durch den radial nach außen abstehenden Nocken (22) gebildet. Letzterer hat zumindest eine Abstützfläche (23) und eine Anlagefläche (24). Nach Figur 5 liegt auf der Abstützfläche (23) der Stempelteller (73) des gespannten Einweg-Injektors über seine Bundfläche (75) auf. Die Abstützfläche (23), die hier die Funktion einer Keilfläche erfüllt, hat die Form eines Kegelstumpfmantels mit einem Spitzenwinkel von 120 Winkelgraden.

Ggf. haben die Druckstäbe (21) oder die Bundfläche (75) zumindest im Kontaktbereich eine keramische Panzerung. Ggf. ist die Bundfläche (75) durch eine z.B. aufgeklebte kegelstumpfmantelförmige Unterlegscheibe verstärkt.

Die Anlagefläche (24) der Nocken (22), vgl. Figur 8, ist Teil eines Konus, dessen maximaler Durchmesser z.B. 3 bis 4 Millimeter größer ist als der Außendurchmesser des Gehäuses (10). Die Anlagefläche (24) kontaktiert bei gespanntem Einweginjektor die Innenwandung (59) des hülsenartigen Auslöseeelements (82). Ggf. hat - zur Minimierung der Flächenpressung - die Anlagefläche (24) eine Krümmung, die der Innenwandung (59) entspricht, vgl. Figuren 5 und 6.

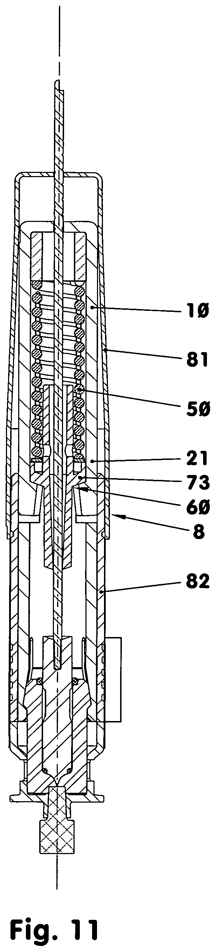

Alternativ zu den Druckstäben (21) können auch Zughaken verwendet werden, vgl. Figur 11. Diese Zughaken (21) sind im oberen Gehäusebereich angeformt. Sie umgreifen - bei gespanntem Federspeicher (50) von oben her den Stempelteller (73) des Kolbenbetätigungsstempels (60). Um beim Betätigen des Injektors dem nach außen schwenkenden Zughaken (21) genug Freiraum zu lassen, ist bei dieser Variante die Auslösekappe (81) bereichsweise kegelstumpfmantelartig ausgeführt.

Unterhalb des Mantelabschnitts (31) befindet sich der Fixierbereich (41) zur Aufnahme der einbaubaren Zylinder-Kolben-Einheit (100), vgl. Figur 7. Der Fixierbereich (41) umfasst z.B. acht parallel zur Mittellinie (5) ausgerichtete Federhaken (42). Die Federhaken (42) haben jeweils einen mindestens zweiflankigen Hintergriff zur ggf. spielfreien Aufnahme der Zylinder-Kolben-Einheit (100). Die einander gegenüber liegenden Flanken (43, 44) des Hintergriffs (42) schließen nach Figur 7 einen Winkel von z.B. 127 Winkelgraden ein. Die untere Flanke (44) hat hierbei einen Kegelwinkel von 45 Winkelgraden, dessen Kegelspitze auf der Mittellinie 5 - in Richtung Düse (106) geblickt - unterhalb der Stirnfläche (17) liegt. Die Länge und die Federrate der Federhaken (42) ist so dimensioniert, dass der Zylinder (101) ohne plastische Verformung der Federhaken (42) eingebaut werden kann.

Um das Gehäuse (10) zusammen mit dem Federelement (50) und dem Kolbenbetätigungsstempel (60) bei der Montage im Auslöseelement (82) verliersicher fixieren zu können, hat das Gehäuse (10) in einem Bereich zwischen den Nocken (22) eine linsenförmige Erhebung (16), vgl. Figur 9, über die das Gehäuse (10) an der Kante (85) des Auslöseelements (82) anliegt.

Der Zylinder (101) ist z.B. ein klarsichtiger, dickwandiger Topf, dessen zumindest bereichsweise zylindrische Außenwandung eine beispielsweise umlaufende Rastrippe (102) trägt, die an den Flanken (43, 44) des Hintergriffs der Federhaken (42) formsteif anliegt. Im Bereich der rückseitigen Stirnfläche des Zylinders (101) befindet sich am oberen Ende der Zylinderinnenwandung (109) ein Bund (119) zur Aufnahme eines Dichtelements (105). Ggf. kann der Bund auch eine radiale ringnutartige Eindrehung sein, die das Dichtelement bereichsweise formschlüssig umgibt. Alternativ oder zusätzlich kann das Dichtelement (105) mit dem Zylinder verklebt sein. Das Dichtelement (105) ist in den Ausführungsbeispielen als O-Ring dargestellt. Es kann aber auch ein Quad-Ring, eine Lippendichtung oder ein anderes gleichwertiges Dichtelement sein.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis 0,5 Millimeter. Diese Bohrung (106) ist ein- bis fünfmal so lang wie ihr Durchmesser. Sie endet in einer zylindrischen oder konischen Ausnehmung (107) der bodenseitigen, äußeren Stirnfläche (103) des Zylinders (101). Die Ausnehmung (107) stellt zumindest annähernd die vorderen ein bis zwei Millimeter eines Luer-Innenkegels dar. Diese Stirnfläche (103) kann zur Erhöhung der Applikationssicherheit zusätzlich mit einem Klebering (104) versehen werden.

Der Zylinder (101) ist beispielsweise aus dem amorphen Thermoplast Cycloolefin-Copolymer (COC) hergestellt. Dieser Werkstoff ist nahezu wasserdampfundurchlässig, was eine dauerhafte Lagerung der Injektionslösung ermöglicht.

In der z.B. zylindrischen oder konischen Bohrung des Zylinders (101) sitzt der stangenlose Kolben (111). Der z.B. aus dem Teflon^{®}-Derivat Tetrafluorethylen/Hexafluorethylen-Copolymer (FEP) hergestellte Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. Die Länge des Kolbens (111) ist so gewählt, dass der eingefahrene Kolben (111), vgl. Figuren 5, 8 oder 9, mindestens einen Millimeter über die hintere Oberkante übersteht. Der mittlere Bereich des Kolbens (111), ist tailliert ausgeführt. Der umlaufend taillierte Bereich hat eine Länge, die ca. 30% der Kolbengesamtlänge entspricht. Der taillierte Bereich hat einen Durchmesser, der 16 bis 20% kleiner ist als der maximale Zylinderinnendurchmesser im Bereich des lösungsaufnehmenden Zylinderinnenraumes (110). Der vordere Übergang, der zwischen dem taillierten Bereich und dem vorderen, also hier untenliegenden, Kolbenbereich liegt, hat z.B. einen Kegelwinkel von 35 bis 40 Winkelgraden. Der andere, hintere Übergang hat einen Kegelwinkel zwischen 35 und 90 Winkelgraden.

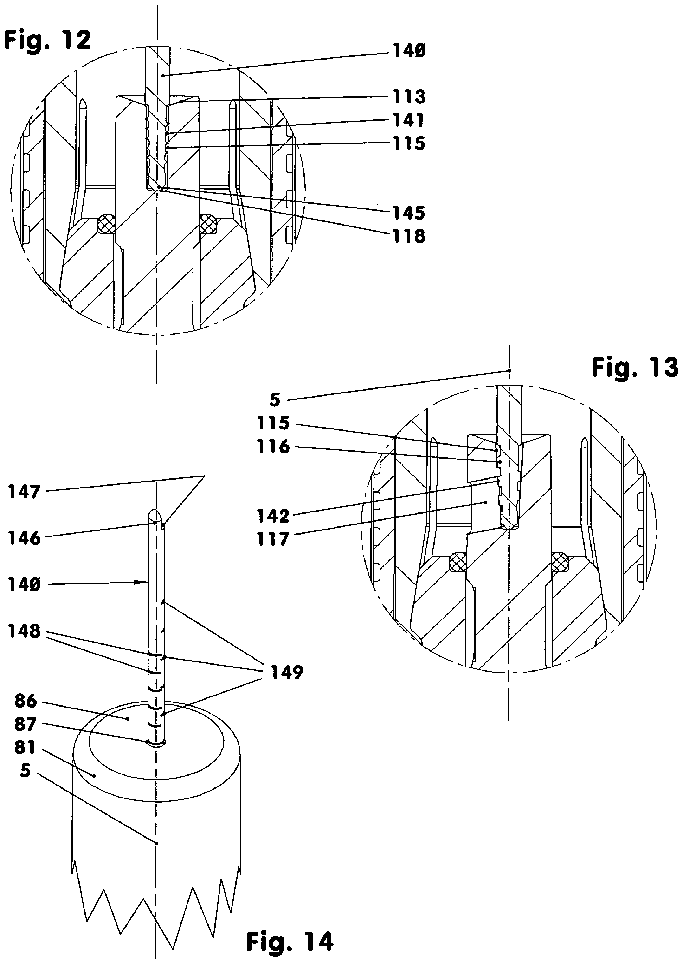

In der rückseitigen, z.B. kegelstumpfmantelförmigen Stirnfläche (113) des Kolbens (111) befindet sich eine zentrische, konische Kolbenausnehmung (115) mit dem Boden (118), vgl. Figur 12, zur Ankupplung der Pumpstange (140). Der Kegelwinkel der Kolbenausnehmung (115) beträgt z.B. ein Winkelgrad. Die Pumpstange (140) hat zum Ankuppeln am Kolben (111) an ihrem unteren Ende u.a. nach Figur 12 ein kegeliges Spitzgewinde (141). Der Kegelwinkel des Spitzgewindes (115) beträgt z.B. sechs Winkelgrade. Der Gewindegang des Spitzgewindes (141) drückt beim Eindrehen der Pumpstange (140) in die Kolbenausnehmung (115) das erforderliche Gegengewinde ein. Der Eindrehvorgang ist beendet, wenn das vordere Ende der Pumpstange (140) mit der schmalen Spitze der kegelstumpfförmigen Stirnseite (145) den Grund (118) kontaktiert.

Die Figur 13 zeigt als Kupplung zwischen der Pumpstange (140) und dem Kolben (111) ein kegeliges Trapezgewinde (142), das in eine Kolbenausnehmung (115) eingreift, in der mindestens ein Teilgang (116) oder eine Nocke angeordnet ist. Der gezeigte Teilgang (116) erstreckt sich im Querschnitt, also normal zur Mittellinie (5), über 30 bis 60 Winkelgrade. Nur über den Teilgang (116) werden die von der Pumpstange (140) auf den Kolben (111) zu übertragenden Axialkräfte weitergeleitet. Aus fertigungstechnischen Gründen befindet sich unterhalb des Teilgangs ein Schieberlangloch (117).

Beide zu Kupplungszwecken verwendeten Sondergewinde (141, 142) benötigen nur geringe Ein- und Ausschraubkräfte. Selbstverständlich können auch andere lösbare Kupplungen verwendet werden, wie z.B. ein Schlüssel/Schlüssellochsystem oder ein einfaches Rastsystem.

Die Pumpstange (140) hat über den größten Teil ihrer Länge z.B. einen gleichbleibenden Querschnitt. Ihr größter Durchmesser beträgt im Ausführungsbeispiel ca. zwei Millimeter. Sie ist z.B. aus einem glasfaserverstärkten Polyamid gefertigt. An ihrem hinteren Ende, das aus der Bohrung (87) der Auslösekappe (81) herausragt, weist sie zwei verschieden geteilte Skalen (148) und (149) auf, vgl. u.a. Figur 14. Die Skala (148) passt zu einer Zylinder-Kolben-Einheit (100), deren Zylinder (101) einen mittleren Innendurchmesser von sieben Millimetern hat, während die andere Skale (149) zu einem Zylinder (101) mit sechs Millimetern Innendurchmesser gehört. Die verschieden großen Zylinder (101) können wahlweise in den Injektor eingesteckt sein.

Beide Skalen (148, 149) haben jeweils horizontale Teilstriche. Diese Teilstriche liegen in Ebenen, die normal zur Mittellinie (5) orientiert sind. Jeder Teilstrich hat beispielsweise die Länge des halben Pumpstangenumfangs. Die Teilstriche einer Skala (148, 149) liegen alle übereinander. Sie können z.B. farbige, schwarz oder weiße Striche sein oder als Kerben in die Pumpstange eingeprägt sein. Statt der Striche können auch Punkte oder Zahlen benutzt werden.

Ggf. kann das hintere Ende der Pumpstange (140) mit einer Struktur versehen sein, z.B. einer Quer- oder Längsrillung, einer Riffelung oder dergleichen. Auch kann der Querschnitt bereichsweise abgeflacht sein oder einen größeren Durchmesser haben, als der Bereich, der sich durch den Kolbenbetätigungsstempel (60) erstreckt.

U.a. nach Figur 9 ist die vordere, dem größeren Zylinder zugeteilte Skala (148) zur Kenntlichmachung der Zuordnung um einen Halbzylinder (146) verlängert. Die beiden unteren, direkt auf dem Niveau des Bodens (86) gelegenen Teilstriche markieren das Nennvolumen der jeweiligen Zylinder (101). Der jeweils oben liegende Teilstrich zeigt an, dass der Kolben (111) vollständig in den Zylinder (101) eingeschoben ist. Jeder andere Teilstrich der Skalen (148, 149) steht z.B. für 0,1 Milliliter.

Zwischen dem Kolben (111) und dem Boden (39) ist der Federenergiespeicher (50) bzw. die Antriebseinheit des Einweginjektors angeordnet.

Der Federenergiespeicher (50) ist eine Schraubendruckfeder, die auf dem Kolbenbetätigungsstempel (60) mit dem Stempelteller (73) angeordnet ist. Er stützt sich am oben liegenden Boden (39) des Gehäuses (10) unter Zwischenschaltung einer Distanzhülse (19) ab. Mittels des Stempeltellers (73) stützt sich der federkraftbelastete Kolbenbetätigungsstempel (60) an den Druckstäben (21) des Gehäuses (10) ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76), der bei einer Betätigung des Einmal-Injektors auf den Kolben (111) wirkt. Der Kolbenschieber (76) hat eine kegelmantelförmige, nach vorn gewölbte Stirnfläche (77), vgl. u.a. Figur 7. Mit dieser Stirnfläche (77) kontaktiert er die komplementär geformte Stirnfläche des Kolbens (111). Beide Kegel haben zumindest annähernd den gleichen Kegelwinkel.

Im Ausführungsbeispiel endet der Kolbenschieber (76) z.B. 2 bis 4 Millimeter oberhalb des Kolbens (111). Der Kolbenbetätigungsstempel (60) hat eine Durchgangsbohrung (63) deren obere Hälfte weitgehend zylindrisch ist, während die untere Hälfte sich nach unten hin konisch verengt. Im Bereich der unteren Stirnfläche (77) ist die Bohrung nur ein bis zwei Zehntel Millimeter größer als der Außendurchmesser der Kolbenstange.

Die Figuren 5 und 7 bis 10 stellen einen Druckstab-Injektor mit einer das Gehäuse fast vollständig umschließenden Auslöseeinheit (80) dar. Das Auslöseelement (82) ist als Teil der Auslöseeinheit (80) hier ebenfalls eine Auslösehülse. Die im Wesentlichen zylindrische, z.B. aus ABS gefertigte, Auslösehülse (82) hat als Stirnfläche die Rücksprungflanke (84) mit der innen liegenden Kante (85). An dem Auslöseelement (82) ist eine Auslösekappe (81) befestigt, die das hintere Ende des Gehäuses (10) umgibt. Die Auslösekappe (81) ist dazu über das hintere Ende des Auslöseelements (82) geschoben.

In Figur 6 ist nur beispielhaft das hintere Ende der Auslösekappe (81) weggelassen. Der hintere Rand der rückwärtig offenen Auslösekappe (81) liegt bei unbetätigtem Injektor - also in der Sperrstellung (8) - auf der Höhe des Gehäusebodens (39) oder darunter.

Unmittelbar oberhalb der Rücksprungflanke (84) befindet sich in der Auslösekappe (81) die Aufweitung (83). Oberhalb der Aufweitung (83) liegt die Auslösekappe (81) gleitfähig an der Außenwandung (13) des Gehäuses (10) an.

Zur Befestigung der Auslösekappe (81) am Auslöseelement (82) hat das Auslöseelement (82) beispielsweise eine Ringnut (56), in die ein Umlaufsteg oder Rastnocken (55) der Auslösekappe (81) eingreift. Ggf. ist die Auslösekappe (81) - zur Erleichterung der Montage - z.B. zweifach bereichsweise längsgeschlitzt.

Im unteren Bereich des Auslöseelements (82) befinden sich in dessen Außenwandung mehrere umlaufende Rillen (57), vgl. Figur 10 oder eine andere vergleichbare Struktur. Die Rillen (57) haben gegeneinander z.B. gleiche Abstände und erstrecken sich über 10 bis 30 Millimetern Länge des Auslöseelements (82).

An der unteren Stirnfläche (58) des Auslöseelements (82) liegt die am Zylinder (101) der Zylinder-Kolben-Einheit (100) zentrierte Verschlusskappe (120) an. Die zumindest annähernd zylindrische Außenfläche der Verschlusskappe (120) hat den gleichen Durchmesser wie die ebenfalls zylindrische Außenfläche des Auslöseelements (82) in der Nähe der Stirnseite (58).

Die Verschlusskappe (120) ist ein Becher, der zumindest das untere Viertel der Zylinder-Kolben-Einheit (100) eng anliegend umgibt. Ein Teil der Verschlusskappe (120) liegt mit ihrem Topfbereich (125) an der zylindrischen Außenwandung des Zylinders (101) und an der unteren Stirnfläche (103) mit dem dort befestigten Klebering (104) an.

Die Verschlusskappe (120) nach den Figuren 5, 6, 9 bis 11 umhüllt nicht nur bereichsweise den Zylinder (101) und liegt dabei an dem Auslöseelement (82) an, sondern stützt sich zusätzlich an der unteren Stirnfläche (17) des Gehäuses (10) ab, vgl. Figur 9. Dazu hat sie in der Nähe ihrer oberen Stirnfläche (121) mehrere, z.B. fünf, an der Innenwandung angeordnete Anlagestege (122). Letztere sind parallel zur Mittellinie (5) ausgerichtet. Die Anlagestege (122) kontaktieren die Außenwandung des Zylinders (101).

Der Topfbereich (125) weist zwei einander gegenüberliegende Fenster (126) auf, vgl. Figur 6. Die Fenster (126) haben eine Breite, die mindestens dem Durchmesser des Kolbens (111) entspricht. Die Unterkante der Fenster (126) - also die Kanten, die dem tellerartigen Fuß (129) am nächsten liegen - sind in der Höhe des Zylinderbodens (108) angeordnet. Mit Hilfe der Fenster (126) lässt sich im Durchlicht u.a. die Blasenfreiheit der Zylinderbefüllung prüfen. Am tellerartigen Fuß (129) ist z.B. ein kegelmantelförmiges Rohrstück als Adapteröffnung (127) angeformt. Die Adapteröffnung (127) hat als Innenwandung zumindest bereichsweise einen Luer-Innenkegel. Nach den Figuren 5, 9 und 10 ist die Adapteröffnung (127) steril verschlossen.

Das zylindrische Auslöseelement (82) ist auf seiner gesamten Länge mit einem Klebeetikett (91) umhüllt. Das Klebeetikett (91) selbst ist z.B. ein mit einem Klebstoff bereichsweise einseitig beschichteter Papier- und/oder Folienstreifen. Der Folienstreifen umgibt z.B. einlagig einmal den Verbund aus Verschlusskappe (120) und Auslöseelement (82). Er besteht als Originalitätsverschluss (90) aus drei separaten Streifen, die jeweils über eine Perforation (96) gegeneinander abtrennbar sind. Der obere Streifen ist das Hauptteil (92), der mittlere Streifen ist eine Abreißbanderole (94) mit einer zwei bis drei Zentimeter langen Abreißfahne (95) und der untere Streifen ist das Kappenteil (93). Das Hauptteil (92) und das Kappenteil (93) tragen eine Klebeschicht, mit der sie an dem Auslöseelement (82) befestigt sind.

Nach Figur 6 wird zum Beladen des Zylinders (101) eine Injektionsnadel (135) benutzt. Sie sitzt über einen Doppelkonusadapter (131) in der Adapteröffnung (127). Letztere bildet zusammen mit der Ausnehmung (107) zumindest annähernd einen Luer-Innenkegel. Zum Beladen wird der Kolben (111) mittels der Pumpstange (140) saugend zurückgezogen. Das hintere Dichtelement (105) verbleibt hierbei ortsfest am Bund (119), auch wenn der taillierte Bereich des Kolbens (111) das Dichtelement (105) passiert. Hat der Kolben (111) seine hintere Stellung erreicht, vgl. Figur 6, liegt das Dichtelement (105) wieder steril radial dichtend am Kolben (111) an. Die Kombination aus den Dichtelementen (114) und (105) gewährleistet auch bei der Pumpbewegung des Kolbens (111) einen sterilen Zylinderinnenraum (110).

Nach dem Beladen und ggf. Entlüften des Zylinders (101) kann zum Zwischenlagern des befüllten Injektors die Adapteröffnung (127) zusammen mit der Ausnehmung (107) mittels eines sterilen Stopfens (128) auch vorn wieder steril verschlossen werden.

Zum Entsichern des Einweg-Injektors wird die Abreißbanderole (94) mit Hilfe der Abreißfahne (95) ringsherum vom Hauptteil (92) und vom Kappenteil (93) getrennt. Die Rillen (57) des Auslöseelements (82) werden sichtbar. Die Verschlusskappe (120) wird nun nach unten vom Zylinder (101) abgezogen, vgl. Figur 7. Nun wird der Injektor auf die Injektionsstelle gesetzt und das hülsenartige Auslöseelement (82) nach unten - in Richtung der Injektionsstelle - geschoben. Die Druckstäbe (21) biegen sich elastisch nach außen in ihre eigentliche Ausgangslage. Hierbei rutschen die Nocken (22) über die Kante (85) nach außen in die Aufweitung (83). Die nun nicht mehr verformten Druckstäbe (21) geben den Kolbenbetätigungsstempel (60) frei, so dass sich der Kolben (111) unter der Wirkung des Federelements (50) ruckartig zum Entleeren des Zylinders (101) nach unten bewegt, vgl. Figur 8. Bei der Vorwärtsbewegung des Kolbens (111) vermindert sich die Kolbenreibung zwischenzeitlich, da das rückwärtige Dichtelement (105) beim Passieren des taillierten Kolbenbereiches nicht bremsend anliegt.

Sollte der Injektor nicht auslösen, so kann die Injektionslösung auch mittels einer Injektionsnadel (135) appliziert werden. Dazu wird die Injektionsnadel (135) mit Hilfe eines Doppelkonusadapters (131) in die Ausnehmung (107) geklemmt. Mit einer in die Ausnehmung (115) des Kolbens (111) eingeschraubten Pumpstange (140), vgl. Figur 6, kann der Injektor wie eine konventionelle Einmalspritze benutzt werden.

Bei dieser Ausführungsvariante können, mit Ausnahme des Federelements (50), alle Bauteile rotationssymmetrisch und/oder zu einer auf der Mittellinie (5) gelegenen Ebene spiegelsymmetrisch aufgebaut sein.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Bei den in den Figuren dargestellten Varianten ist die einzelne Kontaktzone zwischen dem Druckstab (21) und dem Stempelteller (73), als Flächen (23) und (75) ausgeführt, die gleitfähig einander kontaktieren. In einer besonderen Ausgestaltung kann in jeder Fläche (23) der einzelnen Druckstäbe (21) eine Walze gelagert werden, die bei einer Betätigung des Injektors an der Fläche (75) des Stempeltellers wälzgelagert, also reibungsarm, abrollt.

Mit Ausnahme der Federelemente (50, 52), einer ggf. vorhandenen Kolbenplatte und der beispielsweise vorhandenen Lagerwalzen der Stützstäbe (21) sind alle Teile der zuvor beschriebenen Einweg-Injektoren aus Kunststoffen oder kunststoff- bzw. gummiähnlichen Werkstoffen gefertigt.

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil
- 8: Sperrstellung
- 9: Lösestellung, Auslösestellung

- 10: Gehäuse, einteilig
- 13: Außenfläche, zylindrisch
- 16: Erhebung, linsenförmig
- 17: Stirnfläche, vorn, unten
- 19: Distanzhülse

- 21: Druckstäbe, Stützstäbe; Zughaken
- 22: Nocken
- 23: Abstützfläche
- 24: Anlagefläche
- 25: Hintergriffsflanke
- 28: Biegebalken

- 31: Mantelbereich
- 33: Durchbrüche
- 38: Bohrung
- 39: Boden

- 41: Fixierbereich für die Zylinder-Kolben-Einheit
- 42: Federhaken
- 43: Flanke, oben
- 44: Flanke, unten

- 50: Federelement, Schraubendruckfeder, Federenergiespeicher
- 51: Unterlegscheibe, plan
- 52: Federelemente an (21)
- 55: Rastnocken
- 56: Ringnut von (82)
- 57: Rillen von (82)
- 58: Stirnfläche von (82)
- 59: Innenwandung von (82)

- 60: Kolbenbetätigungsstempel
- 62: Führungszapfen
- 63: Bohrung, Durchgangsbohrung

- 73: Stempelteller
- 75: Bundfläche, konisch
- 76: Kolbenschieber
- 77: Kolbenschieberstirnfläche, kegelmantelförmig

- 80: Auslöseeinheit
- 81: Auslösekappe
- 82: Auslöseelement
- 83: Aufweitung
- 84: Rücksprungflanke
- 85: Kante, scharfkantig
- 86: Kappenboden
- 87: Bohrung

- 90: Originalitätsverschluss, Banderole, Sicherungselement
- 91: Klebeetikett
- 92: Hauptteil von (91)
- 93: Kappenteil von (91)
- 94: Abreißbanderole
- 95: Abreißfahne
- 96: Perforationen, Sollbruchstellen

- 100: Zylinder-Kolben-Einheit
- 101: Zylinder
- 102: Rastrippe
- 103: Stirnfläche
- 104: Klebering
- 105: Dichtelement,
- 106: Bohrung, Düse
- 107: Ausnehmung in der Stirnfläche
- 108: Zylinderboden
- 109: Zylinderinnenwandung
- 110: Zylinderinnenraum

- 111: Kolben
- 112: Ringnut
- 113: Stirnseite, hinten; Konus
- 114: Dichtring, Dichtung, Dichtelement
- 115: Kolbenausnehmung, Bohrung
- 116: Teilgang, Nocke
- 117: Schieberlangloch
- 118: Ausnehmungsgrund von (115)
- 119: Bund an (101)

- 120: Verschlusskappe, Klebeversiegelung
- 121: Stirnfläche, hinten
- 122: Anlagestege
- 125: Topfbereich
- 126: Fenster, beidseitig
- 127: Adapteröffnung
- 128: Stopfen
- 129: Fuß, flanschartig

- 131: Doppelkonusadapter
- 135: Injektionsnadel
- 137: Schlauchadapter
- 138: Pumpschlauch

- 140: Pumpstange
- 141: Kegelgewinde, Spitzgewinde
- 142: Sondergewinde, Kegelgewinde
- 145: Stirnseite, kegelstumpfförmig
- 146: Halbzylinder
- 147: Absatz (für kleinen Zylinder)
- 148: Markierungen, Halbkerben für große Zylinder
- 149: Markierungen, Halbkerben für kleine Zylinder

## Patentansprüche

1. Nadelfreier Einweginjektor mit einem Gehäuse (10), in dem oder an dem-jeweils zumindest bereichsweise - mindestens ein mechanischer Federenergiespeicher (50), mindestens eine - zumindest zeitweise wirkstoffbefültbare - Zylinder-Kolben-Einheit (100) mit einem Zylinder (101) mit einer Düse (106) und einem Kolben (111), mindestens ein Kolbenbetätigungsstempel (60) und mindestens eine Auslöseeinheit (80) angeordnet ist,
- wobei der Federenergiespeicher (50) mindestens ein vorgespanntes Federelement umfasst,
- wobei zumindest ein Teil des Kolbenbetätigungsstempels (60) zwischen dem Federenergiespeicher (50) und dem Kolben (111) der Zylinder-Kolben-Einheit (100) positioniert ist und
- wobei der Kolben (111) der Zylinder-Kolben-Einheit (100) - bei vorgespanntem Federelement - über eine hinten aus dem Gehäuse (10) herausragende Pumpstange (140) separat bewegbar ist,
wobei der Zylinder (101) durch ein saugendes zurückziehen des Kolbens (111) mittels der Pumpstange (140) beladen werden kann,
- wobei der Kolbenbetätigungsstempel (60) eine zentrale Bohrung (63) hat, die von der Pumpstange (140) durchquert wird,
**dadurch gekennzeichnet,**
- **dass** die Pumpstange (140) am Kolben (111) oder an einer Kolbenstange des Kolbens (111) lösbar von außen her ankuppelbar ist und
- **dass** der Zylinder (101) der Zylinder-Kolben-Einheit (100) ein im hinteren Bereich der Zylinderinnenwandung (109) angeordnetes Dichtelement (105) aufweist, das den zwischen dem Kolben (111) und dem Zylinder (101) gelegenen Zylinderinnenraum (110) steril verschließt.

2. Einweginjektor gemäß Anspruch 1,
**dadurch gekennzeichnet,**
**dass** die Pumpstange (140) dort, wo sie aus dem Gehäuse (10) herausragt,
eine oder mehrere Skalen (148, 149) aufweist.

3. Einweginjektor gemäß Anspruch 2,
**dadurch gekennzeichnet,**
**dass** die Skalen (148, 149) mehrere Teilstriche oder teilstrichähnliche Markierungen umfassen, wobei ein Teilstrich dem Nennvolumen des Zylinders (101) entspricht, ein Teilstrich ein Nullstrich ist und andere Teilstriche Teilmengen des Nennvolumens darstellen.

4. Einweginjektor gemäß Anspruch 1 oder 2,
**dadurch gekennzeichnet,**
**dass** das hintere Ende der Pumpstange zwei Skalen (148, 149) aufweist, wobei die erste Skala (148) für einen Zylinder (101) mit einem großen Querschnitt und die zweite Skala (149) für einen Zylinder (101) mit einem kleinen Querschnitt vorgesehen ist.

5. Einweginjektor gemäß Anspruch 2,
**dadurch gekennzeichnet,**
**dass** die Teilstriche einer einzelnen Skala (148, 149) jeweils halbkreisförmige übereinander angeordnete Striche oder Kerben sind, wobei die Seite der Pumpstange (140) mit der ersten Skala (148) um einen Halbzylinder (146) verlängert ist.

6. Einweginjektor gemäß Anspruch 1,
**dadurch gekennzeichnet,**
**dass** das Dichtelement (105) radial am Kolben (111) anliegt.

7. Einweginjektor gemäß Anspruch 1,
**dadurch gekennzeichnet,**
**dass** der Kolben (111) im mittleren Bereich tailliert ist.

8. Einweginjektor gemäß Anspruch 1,
**dadurch gekennzeichnet,**
**dass** das Gehäuse (10) mindestens einen Druck-oder Zughaken (21) aufweist, der jeweils im Bereich seines freien Endes mindestens eine Abstützfläche (23) hat, an der der Kolbenbetätigungsstempel (60) anliegt, dass die sperrende Lage des Druck- oder Zughakens (21) durch ein in einer Sperrstellung (8) positioniertes Auslöseelement (82) gesichert ist, dass das Auslöseelement (82) eine Sperrstellung (8) hat, in der es an einer Verschlusskappe (120) gesichert anliegt und dass das Auslöseelement (82) eine Lösestellung (9) hat, die ein seitliches Zurückweichen des Druck- oder Zughakens (21)- unter Freigabe des Kolbenbetätigungsstempels (60) - bewirkt.

9. Einweginjektor gemäß Anspruch 8,
**dadurch gekennzeichnet,**
**dass** der Kolbenbetätigungsstempel (60) an seiner - dem Federelement (50) abgewandten - Stirnfläche zumindest bereichsweise ebene Keilflächen oder bereichsweise einzelne kegelstumpfmantelförmige Flächen (75) aufweist.

10. Einweginjektor gemäß Anspruch 8,
**dadurch gekennzeichnet,**
**dass** der Kolbenbetätigungsstempel (60) zusammen mit jedem einzelnen Druck-oder Zughaken (21) ein Schiebekeilgetriebe bildet, in dem eine axiale Federkraftrichtung in eine radiale Stützkraftrichtung umgelenkt wird.

11. Einweginjektor gemäß Anspruch 8,
**dadurch gekennzeichnet,**
**dass** der einzelne Druck- oder Zughaken (21) jeweils am Gehäuse (10) angeformt ist und einen elastischen Biegebalken (28) darstellt.

## Claims

1. Needle-free disposable injector with a housing (10), in which or on which - respectively at least in certain areas - at least one mechanical spring-energy storage(50), at least one cylinder-piston unit (100) which can be filled at least occasionally with active ingredient and having a cylinder (101) with a nozzle (106) and a piston (111), at least one piston-actuating plunger (60) and at least one actuating unit (80) are arranged,
- whereby the spring-energy storage (50) includes at least one pre-stressed spring-loaded element,
- whereby at least part of the piston-actuating plunger (60) is positioned between the spring-energy storage (50) and the piston (111) of the cylinder-piston unit (100) and
- whereby the piston (111) of the cylinder-piston unit (100) - with a pre-stressed spring element - can be moved separately via a pump rod (140) which, at its rear, protrudes from the housing (10),
- whereby the cylinder (101) can be filled by pulling the piston (111) back with a suction action by means of the pump rod (140),
- whereby the piston-actuating plunger (60) has a central bore (63), through which the pump rod (140) passes,
**characterized**
- **in that** the pump rod (140) can be coupled releasably from the outside to the piston (111) or to a piston rod of the piston (111), and
- **in that** the cylinder (101) of the cylinder-piston unit (100) has a sealing element (105) that is arranged in the rear area of the cylinder inner wall (109) and permits sterile closure of the cylinder interior (110) located between the piston (111) and the cylinder (101).

2. Disposable injector according to Claim 1, **characterized in that** the pump rod (140), at the location where it protrudes from the housing (10), has one or more scales (148, 149).

3. Disposable injector according to Claim 2, **characterized in that** the scales (148, 149) comprise several division marks or division-mark-like markings, where one division mark corresponds to the nominal volume of the cylinder (101), one division mark is a zero mark, and the other division marks represent subquantities of the nominal volume.

4. Disposable injector according to Claim 1 or 2, **characterized in that** the rear end of the pump rod has two scales (148, 149), where the first scale (148) is provided for a cylinder (101) with a large cross section, and the second scale (149) is provided for a cylinder (101) with a small cross section.

5. Disposable injector according to Claim 2, **characterized in that** the division marks of an individual scale (148, 149) are each semicircular marks or notches arranged above one another, where the side of the pump rod (140) with the first scale (148) is lengthened by half a cylinder (146).

6. Disposable injector according to Claim 1, **characterized in that** the sealing element (105) bears radially on the piston (111).

7. Disposable injector according to Claim 1, **characterized in that** the piston (111) is narrowed in the middle area.

8. Disposable injector according to Claim 1, **characterized in that** the housing (10) has at least one compression hook or draw hook (21), which has at least one support surface (23) respectively in the region of its free end, on which the piston-actuating plunger (60) rests, **in that** the locking position of the compression hook or draw hook (21) is secured by an actuating element (82) positioned in a locked position (8) **in that** the actuating element (82) has a locked position (9), in which it rests securely on a sealing cap (120), and **in that** the actuating element (82) has a triggering position (9), which effects lateral retreat of the compression hook or draw hook (21) when the piston-actuating plunger (60) is released.

9. Disposable injector according to Claim 8, **characterized in that** the piston-actuating plunger (60) has on its front end averted from the spring-loaded element (50) at least in certain areas flat wedged faces or in certain areas single frustoconical faces (74, 75).

10. Disposable injector according to Claim 8, **characterized in that** together with every single compression hook or draw hook (21) the piston-actuating plunger (60) forms a spline gear, in which an axial resilient force direction is deflected in a radial support force direction.

11. Disposable injector according to Claim 8, **characterized in that** the single compression hook or draw hook (21) is formed respectively on the housing (10) and represents an elastic flexional beam (28).

## Revendications

1. Injecteur à usage unique sans aiguille, comprenant un boîtier (10) dans lequel ou sur lequel sont disposés - à chaque fois au moins en partie - au moins un accumulateur d'énergie à ressort mécanique (50), au moins une unité cylindre-piston (100) - pouvant être remplie au moins temporairement de substance active - avec un cylindre (101) avec une buse (106) et un piston (111), au moins un poinçon d'actionnement de piston (60) et au moins une unité de déclenchement (80),
- l'accumulateur d'énergie à ressort (50) comprenant au moins un élément de ressort précontraint,
- au moins une partie du poinçon d'actionnement de piston (60) étant positionnée entre l'accumulateur d'énergie à ressort (50) et le piston (111) de l'unité cylindre-piston (100) et
- le piston (111) de l'unité cylindre-piston (100) - lorsque l'élément de ressort est précontraint - pouvant être déplacé séparément par le biais d'une tige de pompe (140) faisant saillie à l'arrière hors du boîtier (10), le cylindre (101) pouvant être chargé au moyen de la tige de pompe (140) par une rétraction par aspiration du piston (111),
- le poinçon d'actionnement de piston (60) présentant un alésage central (63) qui est traversé par la tige de pompe (140),
**caractérisé en ce que**
- la tige de pompe (140) peut être accouplée au piston (111) ou à une tige de piston du piston (111) de manière amovible depuis l'extérieur et
- le cylindre (101) de l'unité cylindre-piston (100) présente un élément d'étanchéité (105) disposé dans la région arrière de la paroi intérieure du cylindre (109), qui ferme de manière stérile l'espace intérieur d'un cylindre (110) entre le piston (111) et le cylindre (101).

2. Injecteur à usage unique selon la revendication 1,
**caractérisé en ce que**
la tige de pompe (140) présente une plusieurs échelles (148, 149) à l'endroit où elle sort du boîtier (10).

3. Injecteur à usage unique selon la revendication 2,
**caractérisé en ce que**
les échelles (148, 149) comprennent plusieurs traits de marquage ou des marquages similaires à des traits de marquage, un trait de marquage correspondant au volume nominal du cylindre (101), un trait de marquage étant un trait de point zéro et les autres traits de marquage constituant des quantités partielles du volume nominal.

4. Injecteur à usage unique selon la revendication 1 ou 2,
**caractérisé en ce que**
l'extrémité arrière de la tige de pompe présente deux échelles (148, 149), la première échelle (148) étant prévue pour un cylindre (101) ayant une grande section transversale et la deuxième échelle (149) étant prévue pour un cylindre (101) ayant une petite section transversale.

5. Injecteur à usage unique selon la revendication 2,
**caractérisé en ce que**
les traits d'une échelle individuelle (148, 149) sont à chaque fois des traits ou des crans disposés les uns au-dessus des autres en forme de demi-cercle, le côté de la tige de pompe (140) avec la première échelle (148) étant prolongé d'un demi-cylindre (146).

6. Injecteur à usage unique selon la revendication 1,
**caractérisé en ce que**
l'élément d'étanchéité (105) s'applique radialement contre le piston (111).

7. Injecteur à usage unique selon la revendication 1,
**caractérisé en ce que**
le piston (111) est aminci dans la région centrale.

8. Injecteur à usage unique selon la revendication 1,
**caractérisé en ce que**
le boîtier (10) présente au moins un crochet de pression ou de traction (21) qui présente à chaque fois dans la région de son extrémité libre au moins une surface de support (23) contre laquelle s'applique le poinçon d'actionnement de piston (60), **en ce que** la position de blocage du crochet de pression ou de traction (21) est fixée par un élément de déclenchement (82) positionné dans une position de verrouillage (8), **en ce que** l'élément de déclenchement (82) présente une position de verrouillage (8), dans laquelle il s'applique de manière fixée contre un capuchon de fermeture (120) et **en ce que** l'élément de déclenchement (82) présente une position de libération (9), qui provoque un recul latéral du crochet de pression
ou de traction (21) en libérant le poinçon d'actionnement de piston (60).

9. Injecteur à usage unique selon la revendication 8,
**caractérisé en ce que**
le poinçon d'actionnement de piston (60) présente, au niveau de sa surface frontale opposée à l'élément de ressort (50), au moins en partie des surfaces de clavette planes ou en partie des surfaces (75) en forme d'enveloppe tronconique individuelles.

10. Injecteur à usage unique selon la revendication 8,
**caractérisé en ce que**
le poinçon d'actionnement de piston (60) forme, conjointement avec chaque crochet de pression ou de traction individuel (21), un mécanisme à cale coulissante dans lequel une direction de force de ressort axiale est déviée dans une direction de force de support radiale.

11. Injecteur à usage unique selon la revendication 8,
**caractérisé en ce que**
le crochet de pression ou de traction individuel (21) est à chaque fois formé sur le boîtier (10) et constitue une poutre flexible élastique (28).
